(19) Europäisches Patentamt / European Patent Office / Office européen des brevets

(11) **EP 4 109 333 B1**

(12) **EUROPEAN PATENT SPECIFICATION**

(45) Date of publication and mention
of the grant of the patent:
**12.03.2025 Bulletin 2025/11**

(21) Application number: **22179854.9**

(22) Date of filing: **20.06.2022**

(51) International Patent Classification (IPC):
**G01N 15/1433** *(2024.01)*    **G01N 33/58** *(2006.01)*
**G06V 20/69** *(2022.01)*    **G01N 15/14** *(2024.01)*
**G06K 19/06** *(2006.01)*    **G01N 33/533** *(2006.01)*

(52) Cooperative Patent Classification (CPC):
**G01N 15/1433; G01N 33/582; G06V 20/698**

(54) **METHOD FOR ANALYSING BIOLOGICAL SAMPLES**

VERFAHREN ZUR ANALYSE BIOLOGISCHER PROBEN

PROCÉDÉ D'ANALYSE D'ÉCHANTILLONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2021 PCT/EP2021/066645**
**28.08.2021 PCT/EP2021/073819**
**23.12.2021 PCT/EP2021/087551**
**23.12.2021 PCT/EP2021/087558**

(43) Date of publication of application:
**28.12.2022 Bulletin 2022/52**

(73) Proprietor: **Leica Microsystems CMS GmbH**
**35578 Wetzlar (DE)**

(72) Inventors:
• **Alsheimer, Soeren**
**60320 Frankfurt am Main (DE)**
• **Bradl, Joachim**
**68519 Viernheim (DE)**
• **Walter, Kai**
**69198 Schriesheim (DE)**

(74) Representative: **Schaumburg und Partner Patentanwälte mbB**
**Mauerkircherstraße 31**
**81679 München (DE)**

(56) References cited:
**US-A1- 2018 030 504    US-A1- 2021 166 380**

• **GARAPATI HITA SONY ET AL: "Predicting subcellular localization of proteins using protein-protein interaction data", GENOMICS, ACADEMIC PRESS, SAN DIEGO, US, vol. 112, no. 3, 14 January 2020 (2020-01-14), pages 2361 - 2368, XP086089674, ISSN: 0888-7543, [retrieved on 20200114], DOI: 10.1016/ J.YGENO.2020.01.007**

**Description**

Technical field:

The present invention relates to a method for analysing a biological sample with a plurality of analytes, in particular, by optical readout.

Background:

The organs of metazoans such as vertebrates or humans are made up of tissues that themselves consists of cells. Ultimately, these cells are all derived from the same egg cell and share essentially the same genome. It is estimated that the human genome contains on the order of 30,000 genes, which encode on the order of around 22,000 proteins. Surprisingly, it appears that the number of protein-coding genes in mice and humans is roughly the same and that only around 1% of the genes in mice and human genomes do not have an ortholog in the other species. For comparison the number of proteins encoded by the yeast genome is on the order of 6,250 proteins. Thus, while there is clearly an expansion of the number of genes from unicellular eukaryotes to metazoans, the complexity increase within the metazoan clade is largely not accompanied by a proportional increase in the number of genes.

The complexity of humans appears to be attributable to a more complex regulation of genes as well as to a significantly increased use of alternative splicing. The basic building blocks are apparently reused over-and-over again during metazoan evolution. This is important for the present disclosure as it suggests that observations concerning the expression of certain genes in certain cell types of the mouse or other vertebrates can be to a large degree transferred to human tissues and vice versa. This notion is well accepted in the scientific community and justifies the use of model systems for basic and translational research.

The organs of humans or animals develop during embryonic and foetal development from multiple tissues, which themselves develop from cellular lineages, which are set up early in development. These cellular lineages, such as the hematopoietic lineage, which gives rise to the cells of the blood or cells of the immune system, generally contain stem cells that allow the maintenance of the corresponding cell lineage during adult life.

As development proceeds the differentiation and maturation of cells, tissues, and organs proceeds, which changes both the gene expression programs that become activated and repressed by epigenetic memory. The phenotype of a given cell at a given moment in time can be seen as the output of all the interactions of its molecular constituents and can be regarded as a certain position in the cell state space.

Recently, the advent of single cell RNA sequencing and single multi-omics technologies has made it feasible to access and map this cell state space, with the intent to generate cell atlases, that can identify all cell types in the human body and serve as a reference database comparable to a "periodic table of cell types" (ref. Human Cell Atlas, MOCA). While some of these initiatives try to map the physiological cell state space, others are trying to map disease states, for example the HTAN initiative.

A particular focus of application of these databases has been in the field of sample imaging. For example, next generation microscopy (NGM) is emerging from the convergence of cytometry, microscopy, and single cell/spatial biology technologies. NGM solutions need to provide plexing levels which are sufficiently high in order to measure a sufficient number of datapoints for a particular cell or tissue, to be able to discern the phenotype of the cell, for example. Current spatial biology solutions are using FISH-based methods and combinatorial encoding to offer plexing levels in the range of several hundreds to several thousands on the RNA level. While this is significantly higher than what conventional microscopy provides in terms of plexing, there are still important drawbacks of current methods such as MERFISH, SeqFISH+, or the approach of Nanostring's CosMx imager, in that they require a spot-based readout and many rounds of staining and imaging. Thus, despite these advances, there remains a need for improved analysis methods that provide high plexing levels.

Document Garapati et al., 2020 (Predicting subcellular localisation of proteins using protein-protein interaction data) discloses a method for predicting the localisation of proteins in cells based on protein-protein interaction data.

Document US 2021/166380 A1 discloses the analysis of tissue sections to identify malign structures.

Summary

It is an object to provide a method that allows efficient and fast analysis of a biological sample with a plurality of analytes, in particular, based on an optical readout.

The aforementioned object is achieved by the subject-matter of the independent claim 1. Advantageous embodiments are defined in the dependent claims.

Next generation microscopy (NGM) solutions are built to generate probabilistic mathematical models of the sample, which may represent the sample as a multi-scale multilayer graph for example. The concept disclosed in the documents PCT/EP2021/066645 and PCT/EP2021/073819 or the document PCT/EP2021/063310, introduced a new solution to the problem of attaining very high plexing levels, without necessitating spot-based readout. In this context, the content of the following documents are relevant for the present invention as well: PCT/EP2021/087551 and PCT/EP2021/087558. In contrast to other methods, PCT/EP2021/066645 and PCT/EP2021/073819 can decode readout volumes in which multiple target molecule species, e.g. protein L, protein K, mRNA N, metabolite Y....protein Z, are marked with markers comprising combinatorial labels, i.e. that comprise a combination of multiple dye species, wherein each dye species may be present as a single or as a plurality of dye molecules (e.g. label K = 1 molecule dye A, 5 molecules dye B, 30 molecules dye H).

For example, the methods in PCT/EP2021/066645 and PCT/EP2021/073819 can be used with a set of dyes comprising 50 dyes, from which combinatorial labels can be generated, in this case the method can be used to assess 1,000 markers in 3 rounds of imaging even if up to 10 different target molecule species that are marked by markers may be contained within the same readout volume. The method disclosed in the present document may be used to significantly improve the decoding power of the aforementioned and similar imaging methods and algorithms and enables the decoding even if a much higher number of different analytes that are marked by markers is present in the same readout volume.

A method for analysing a biological sample with a plurality of analytes, comprises the following steps: Determining information about the biological sample and its analytes, the analytes being marked by a plurality of markers; Generating a probabilistic model of the distribution of the analytes within the biological sample based on the determined information; Generating at least one optical readout of the biological sample; Determining the presence, in particular, the location and/or co-location, of at least one analyte in the readout based at least partially on the probabilistic model of the distribution of the analytes within the biological sample.

In particular, as is known in the state of the art, determining the presence of at least one analyte may be based on the optical readout and the optical signals detected from the respective markers used to label the analytes. However, when analysing a larger number of analytes and when simultaneously using a large number of markers with combinations of dyes, the optical signals may interfere with each other and be difficult to unambiguously distinguish from one another. In order to increase the ability to unambiguously distinguish these optical signals, the presence of the analytes may be determined based partially on the probabilistic model. To that end, the probabilistic model comprises information on the likelihood that a particular analyte may be found in a particular location of the biological sample and/or the probabilistic model comprises information on the likelihood that a particular analyte may be found in (close) proximity with another particular analyte. For example, a membrane protein may have a high probability to be in close proximity to another membrane protein. Thus, the localisation/co-localisation probabilities are used to inform the process of distinguishing between several optical signals of markers that mark the analytes, especially when these analytes are in close proximity. The underlying assumption is, that biological samples are highly structured or highly correlated. The presence of a protein with known nucleolar localisation, for example, suggests that the corresponding pixel of the optical readout is part of a set of neighbouring pixels belonging to the nucleolus and thus it is more probable to find other proteins with known nucleolar localisation in the same or neighbouring pixels. Likewise, it is possible but less likely to find proteins with known cytosolic localisation or proteins of the plasma membrane in the nucleolus.

The aforementioned method may be particularly applicable to the (microscopy) method disclosed in the documents PCT/EP2021/066645 and PCT/EP2021/073819, in order to increase speed and efficiency of analysing the optical readouts obtained by the methods in these documents.

The biological sample may in particular be a tissue sample from a biopsy, that may be prepared by sectioning. In particular, each analyte may be marked by a respective marker, that means that the marker is attached to one particular analyte. The analyte may, for example, be a particular protein of interest within a cell of the biological sample. That protein of interest may be of interest because it is associated with a particular pathological state or because it marks a particular location within a cell such as the cell membrane. The marker generally may comprise an affinity reagent, that attaches only to one particular analyte, a dye, that may be readout optically, and an optional linker that connects the dye to the affinity reagent. In particular, when generating the optical readout, the dye may be excited at an excitation wavelength and emitted light may be readout at an emission wavelength. The information may be determined before and/or after generating the optical readout. Preferably, the information at least includes spatial information of the analytes, that means for example, information about the location of analytes within the biological sample (or within a particular level of the biological sample) and/or the co-localisation of at least two analytes with each other. For example, the information may include co-localisation information for a particular membrane protein with another membrane protein.

Thus, in one aspect this provides a method to use *a priori* information on the presence of biological features such as tissues, tissue modules, cells, organelles, vesicles, cellular structures, proteins, mRNA, genes. In particular, the present disclosure proposes to use *a priori* information derived from single cell RNA sequencing and single cell multi-omics efforts as well as information on protein complexes, protein subcellular localisation as well as protein-protein interaction to be used to calculate probability models or maps that are anchored to features of cells (such as cell membranes or cell nucleus) or tissues (such as for example certain cell types, e.g. T-cells) in biological samples. These probability models essentially provide gene-specific probabilities on a pixel-per-pixel basis in images being acquired from a respectively labelled sample in the sense of Bayesian statistics or weights in the sense of graphs that are used to boost the decoding strength of decoding algorithms as proposed in PCT/EP2021/066645 and PCT/EP2021/073819, for example. The underlying assumption here is, that biological samples are highly structured or highly correlated. The presence of a protein with known nucleolar localisation, for example, suggest that the corresponding pixel is part of the set of pixels belonging to the nucleolus and thus it is more probable to find other proteins with known nucleolar localisation in the same or neighbouring pixels. Likewise, it is possible but less likely to find proteins with known cytosolic localisation or proteins of the plasma membrane in the nucleolus. Similarly, the presence of markers that have a high sensitivity and high specificity for a given cellular lineage and/or a given cell type as well as a combination of such markers, if detected can be used to assign a cell to a certain cell type in a corresponding ontology. The same can be said about cell states. In this way, cells in the sample can be progressively classified as more and more markers are being measured, this allows more reliable prediction of gene expression programs, epigenetic marks, signalling network states etc. to be made, which in turn further aid in decoding and therefore accelerate measurement of further markers.

It is important to note, that a priori information in the sense of this document may derive from measurement or prediction. A priori information in the sense of this document may be obtained by any of the following retrieving from a database, inferring from data retrieved from a database, inferring from measurements of the sample at the same or a different location. A priori information in the sense of this document may commonly include information from multiple modalities and data-layers, which are not necessarily identical to the data layer or modality generated in the subsequent analysis. For example, DNA sequence data from whole genome sequencing, epigenetic marks from ATACseq, and transcriptome data may be used to measure gene expression profiles for cell types present in the sample using single cell multi-omics technologies. Alternatively or in addition, cell type composition and cell type-specific gene expression signatures may be imputed from bulk whole exome sequencing using tools such as CIBERSORTx. In particular machine and deep learning may be used to analyse such data sets to generate predictions on the presence of certain cell types and cell states in the sample as well as on associated gene and protein expression signatures as well as on protein distribution, localisation, and protein complex formation and protein-protein interaction in the respective cell types. This a priori information is then correlated to at least a first image of the sample which may be a label-free image or an image bearing at least a first set of markers. In this way, cells in the biological sample will be assigned a complement of a priori information such as prediction on cell type, set of cell states, gene/protein expression profile, subcellular localisation of proteins, and so on. The match of an actual cell type or cell state with the prediction is determined or called based on a measure of statistical confidence and/or the presence of marker sets, which may be defined by the user or by machine learning tools such as CellTypist.

This greatly facilitates decoding as exemplified by the following: in case it is desired to image a tissue section (the biological sample) of the gut, which contains healthy colon tissue and colon tumour tissue, and a pre-analysis was already performed using information from the Human Cell Atlas as well as HTAN atlas and using single cell RNA sequencing and UMAP clustering (Uniform Manifold Approximation and Projection) on a part of the same biopsy. Then a priori, we would have a very good approximation of the different cell types, cell states, as well their gene or protein expression signatures on the single cell level of the biological sample, before imaging the sample. The analytes in this case may be particular mRNAs or particular proteins, with different cell types, cell compartments or cell states having a characteristic composition of these analytes. This information about the analytes and the different cell types, cell states and so on may be the basis to generate the probabilistic model of the distribution of analytes within the biological sample.

Next it would be possible to use the methodology of documents PCT/EP2021/066645 and PCT/EP2021/073819 to image +1,000 markers in the corresponding tissue section, and in the n-th round of imaging there may be many readout volumes in different cells for which an unambiguous decoding is not possible owing to fact too many analytes are marked by markers at the same time. Suppose these cells are, however, different cells, for example, some of the cells are cells of the intestinal epithelium and healthy, whereas others are cells of the immune system or stroma and again others are cells of the tumour. Then the method of the present document enables making very good predictions on the compositions of these cells based on the a priori knowledge of the probabilistic model. It may be possible, for instance, to exclude a large number of possible markers marking analytes of an intestinal cell, because they point to proteins, that are usually not expressed in intestinal cells but in cells of the immune system, for example.

Preferably, the information about the samples is determined from at least one of the following sources: generic databases or data generated from the biological sample. The database may be at least one of HubMAP, Human Cell Atlas, HTAN, and LocDB, for example. These databases contain previously collected information about biological systems. The type of information contained in these databases may alternatively or in addition collected from the actual biological sample, for example, by determining the transcriptome of the sample. This enables determining a wide variety and/or very specific information about the biological sample.

Preferably, the information about the sample comprises at least one of the following types: genomic, transcriptomic, proteomic, metabolomic, interactomic, localisomic (this information pertains to localisation or co-localisation of target molecules within the sample or within a cell of the sample), or epigenomic. This enables determining a wide variety and/or very specific information about the biological sample.

Preferably, the information about the sample is determined for at least one of the following levels: subcellular, organellular, cellular, or tissular. This enables determining a wide variety and/or very specific information about the biological sample.

It is particularly preferred, when the information about the sample is at least determined prior to generating the one optical readout. This enables a fast and efficient analysis of the biological sample, since already available information is used to analyse the sample. In a particular embodiment, the information may in addition be determined after generating the one optical readout, in particular, at least partially from the one optical readout of the biological sample.

It is particularly preferred, when the at least one optical readout is segmented in order to determine the information about the sample from the segmented optical readout. In this case, the optical readout is preferably an image. The segmentation enables determining features of the biological sample, such as individual cells, cell types, structures of a cell, or structures of a tissue. Information based on the segmentation may be combined with information from databases, for example, when generating the probabilistic model and/or when determining the presence of an analyte. This enables generating a particularly detailed probabilistic model and accurately determining the presence of the analyte.

It is preferably, when the information about the sample is determined from the optical readout pixel-per-pixel. The probabilistic model may thus comprise pixel-per-pixel probabilities about the likelihood of the presence of an analyte at a particular pixel derived from the information. This enables generating a particularly detailed probabilistic model and accurately determining the presence of the analyte.

Preferably, the probabilistic model is modified based on information about the biological sample determined from the at least one optical readout, preferably, the probabilistic model is iteratively modified based on information about the biological sample determined from a plurality of iteratively generated optical readouts. For example, initially the probabilistic model may be generated prior to generating the optical readout, after generating the initial optical readout and determining information from the optical readout, the probabilistic model is modified based on the information from the optical readout. This enables iterating the probabilistic model and generating a particularly detailed probabilistic model and accurately determining the presence of the analyte.

Preferably, at least one further optical readout is generated and the analytes are marked by a different plurality of markers for the further optical readout. This enables iterative staining or marking of the analytes. The further plurality of markers, in particular, differs from the plurality of markers by their combinations of dyes. In a particularly preferred embodiment, each combinations of dyes attached to one of the affinity reagents only comprises a single dye. Thus, each marker comprises a single dye; in between readouts the analytes are iteratively marked by markers with different single dyes attached. In yet another embodiment, the combinations of dyes comprise several dyes. In this case, at least one dye of the several dyes of each combination of dyes is changed to generate the different markers between readouts.

Preferably, at least the plurality of markers is based on the probabilistic model of the distribution of the analytes. In particular, determining the particular combinations of affinity reagents and dyes or combination of dyes or first mapping, may be based on the probabilistic model. Preferably the determination of the plurality of markers is determined prior to performing the optical readout. This may also be performed iteratively, where a further plurality of markers is generated based on the optical readouts.

For example, the markers may be chosen such that only those analytes are marked that are of particular interest, or the markers may be chosen such that only those analytes are marked that are predicted by the probabilistic model to be sufficiently far apart to allow easy identification of a particular marker in the optical readout. This enables particularly efficient analysis of the biological sample.

Preferably, the at least one optical readout is generated by means of a readout device such as a microscope or a flow cytometer. This enables accurately generating the optical readout.

Preferably, generating the optical readout comprises, the steps also detailed in documents PCT/EP2021/066645 and PCT/EP2021/073819: Providing the plurality of markers comprising a plurality of affinity reagents, each affinity reagent configured to attach to one of the analytes, and a first plurality of combinations of dyes, each combination of dyes being unique within the first plurality of combinations of dyes and each combination of dyes comprising at least two dyes having different characteristics for at least one of: excitation and emission, wherein each one of the unique combinations of dyes is attached to an associated affinity reagent according to a first mapping. Directing excitation light at the sample, the excitation light having characteristics for exciting at least the at least two dyes having different characteristics for at least one of: excitation and emission. And generating at least one first optical readout from emission light emitted by the excited dyes. This enables detecting the presence of analytes in a sample particularly accurately. In particular, by directing excitation light having characteristics for exciting dyes having different excitation and/or emission characteristics, the readout generated can contain information allowing for the determination of the presence of a greater number of analytes per readout than is possible using known methods.

Preferably, the method may be further defined in that each unique combination of dyes in the first plurality of combinations of dyes is attached to only one affinity reagent, such that no unique combination of dyes is associated with more than one affinity reagent in the first mapping. In this way, the detection of a unique combination of dyes within a readout can, with confidence, be used to determine that an analyte is present in the sample. It may also be said that the mapping of the plurality of combinations of dyes to affinity reagents is at least injective, preferably bijective.

Preferably, the method further comprises at least one of, the steps also detailed in applications PCT/EP2021/066645 and PCT/EP2021/073819: Deactivating at least one of the dyes in the first plurality of combinations of dyes; Removing the attachment between at least one affinity reagent and at least one of the combinations of dyes; Removing the attachment between at least one affinity reagent and at least one of the analytes; And waiting longer than a fluorescence lifetime of at least one of the dyes in the first plurality of combinations of dyes; And repeating steps i) to iii) for generating the optical readout for a second, different, plurality of combinations of dyes or for the first plurality of combinations of dyes according to a second, different, mapping.

This enables generating more information that pertains to the same sample. A single combination of dyes and/or single mapping may not produce a readout from which the presence of any or all analytes can be determined. It is therefore desirable to obtain more information relating to the original sample. The method may therefore deactivate, or allow to deactivate, at least one of the dyes or attachments, such that the (further) readout generated when steps i) to iii) are run again will be different to the original (initial) readout. The further readout having new information relative to the initial can then be used to determine the presence of the further analytes and/or to generate or adjust the probabilistic model.

Preferably the method further comprises providing at least one dye and/or dye combination of dyes for the second plurality of combinations of dyes and/or rules for the second mapping, based on the at least one first optical readout. This enables reduction of the number of iterations, thereby accelerating the method.

Preferably, the method further comprises iteratively repeating the steps of the previous two paragraphs for at least one of: a number of pluralities of combinations of dyes; or a number of mappings, until all affinity reagents attached to analytes in the sample are determined. In this way, the method determines the presence of all analytes in the sample.

Preferably, the optical readout comprises at least one readout volume and wherein the determination of the presence of at least two different analytes in the readout volume is based at least partially on the probabilistic model of the distribution of the analytes within the biological sample.

Terms:

**"Sample":** In the sense of this document "sample" refers to a biological sample which may also be named a biological specimen including, for example blood, serum, plasma, tissue, bodily fluids (e.g. lymph, saliva, semen, interstitial fluid, cerebrospinal fluid), feces, solid biopsy, liquid biopsy, explants, whole embryos (e.g. zebrafish, Drosophila), entire model organisms (e.g. zebrafish larvae, Drosophila embryos, C. elegans), cells (e.g. prokaryotes, eukaryotes, archea), multicellular organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document "sample" further refers to a volume surrounding a biological sample. For example in assays, where secreted proteins like growth factors, extracellular matrix constituents are being studied the extracellular environment surrounding a cell up to a certain assay-dependent distance, is also referred to as the "sample". Specifically, affinity reagents brought into this surrounding volume are referred to in the sense of this document as being "introduced into the sample".

**"Affinity reagent":** In the sense of this document the term "affinity reagent" may in particular be an antibody, a single-domain antibody (also known as nanobody), a combination of at least two single-domain antibodies, an aptamer, an oligonucleotide, a morpholino, a PNA complementary to a predetermined RNA, DNA target sequence, a ligand (e.g. a drug or a drug-like molecule), or a toxin, e.g. Phalloidin a toxin that binds to an actin filament. In the sense of this document an affinity reagent is configured to bind a target molecule or to an analyte with a certain affinity and specificity such that it can be said that the affinity reagent is substantially specific to the target molecule or predetermined target structure. In the sense of this document "plurality of affinity reagents" ($S_2$) contains the affinity reagents ($a_1$, $a_2$, $a_3$,...$a_n$), which are configured to specifically bind to a predetermined target structure within the biological sample or to a predetermined chemical compound or to a predetermined chemical element or to an analyte. At least some of the affinity reagents from the plurality of affinity reagents ($A$) are "introduced to the sample" such that the affinity reagents can attach to the respective predetermined target structure within the sample. In this context and in the sense of this document and as described above "introduced to the sample" may refer to being physically introduced into the volume of the sample or into a volume surrounding and assigned to the sample. An example of the latter case may be assays for secreted molecules for instance, which are best assessed in the extracellular space where they might be outside of the sample, but within a certain spatial context or vicinity of the sample.

**"Analyte":** In the sense of this document "analyte" refers to a predetermined target structure, a target molecule or a target structure, which may for example be a protein (e.g. a certain protein), an RNA sequence (e.g. the mRNA of a certain gene), a peptide (e.g. somatostatin), a DNA sequence (e.g. the a genetic locus or element), a metabolite (e.g. lactic acid), a hormone (e.g. estradiol), a neurotransmitter (e.g. dopamine), a vitamin (e.g. cobalamine), a micronutrient (e.g. biotin), a metal ion (e.g. metal and heavy metal ions like Cd(II), Co(II), Pb(II), Hg(II), U(VI)).

**"Dye":** In the sense of this document the terms "fluorescent dye", "fluorophore", "fluorochrome", "dye" are used interchangeably to denote a fluorescent chemical compound or structure and can be in particular one of the following: a fluorescent organic dye, a fluorescent quantum dot, a fluorescent dyad, a fluorescent carbon dot, graphene quantum dot or other carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), derivatives, squaraine rotaxane derivatives, naphthalene, coumarin, oxadiazole, anthracene (an-thraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups designated commercially available fluorescent dyes, which may include dyes belonging to different chemical families CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Inter-chim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA Bio-Medicals), Quasar and Cal Fluor dyes (Biosearch Technologies), SureLight Dyes (Columbia Biosciences), Vio Dyes (Milteny Biotec) (list modified from: https://en.wikipedia.org/wiki/Fluorophore). From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Superfolder GFP, mCherry, mPlum) are meant by the term "fluorescent dye" in the sense of this document. Further from the group of fluorescent proteins the term "fluorescent dye" in the sense of this document may include fluorescent proteins, whose absorbance or emission characteristics change upon binding of ligand for example BFPms1 or in response to changes in the environment for example redox-sensitive roGFP or pH-sensitive variants. Further from the group of fluorescent proteins the term "fluorescent dye" in the sense of this document may include derivative of cyanobacterial phycobiliprotein small ultra red fluorescent protein smURFP as well as fluorescent protein nanoparticles that can be derived from smURFP. An overview of fluorescent proteins can be found in Rodriguez et al. 2017 in Trends Biochem Sci. 2017 Feb; 42(2): 111-129. The term "fluorescent dye" in the sense of this document may further refer to a fluorescent quantum dot. The term "fluorescent dye" in the sense of this document may further refer to fluorescent carbon dot, a fluorescent graphene quantum dot, a fluorescent carbon-based nanostructure as described in Yan et al. 2019 in Microchimica Acta (2019) 186: 583 and Iravani and Varma 2020 in Environ Chem Lett. 2020 Mar 10 : 1-25. The term "fluorescent dye" in the sense of this document may further refer to a fluorescent polymer dot (Pdot) or nanodiamond. The term "fluorescent dye" in the sense of this document may further refer to a fluorescent dyad, for example a dyad of a perylene antenna and a triangelium emitter as described in Kacenauskaite et al. 2021 J. Am. Chem. Soc. 2021, 143, 1377-1385.

The term "fluorescent dye" in the sense of this document may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned.

The term "fluorescent dye" in the sense of this document may further refer to a FRET-pair having at least one fluorescent dye as FRET donor and at least one fluorescent dyes as a FRET acceptor, or a FRET-triple, which is used to generate a three component Förster resonance energy transfer. In particular, the FRET-pair or FRET-triplet is connected by a complementary linker or by a linking element.

The term "fluorescent dye" in the sense of this document may further refer to a FRET n-tupel of physically connected dyes.

**"Plurality of combinations of dyes"** ($S_1$): In the sense of this document the term "Plurality of combinations of dyes" ($S_1$) refers to the plurality of combinations of dyes for which, each combination of dyes ($s_1$, $s_2$, $s_3$,...$s_n$) is unique within the plurality of combinations of dyes ($S_1$), each combination of dyes ($s_1$, $s_2$, $s_3$,...$s_n$) comprises at least two different dyes ($|s|$ >=2); wherein the plurality of combinations of dyes ($S_1$) is composed such that each dye ($y_1$, $y_2$, $y_3$,...$y_\sigma$) in the plurality of combinations of dyes ($S_1$) can be readout by a readout device; wherein dyes can be separated by a readout device into channels; each channel corresponding to one of the dyes ($\mathbf{y}_1$, $\mathbf{y}_2$, $\mathbf{y}_3$....$\mathbf{y}_\sigma$).

**"Marker":** In the sense of this document "marker" is used to denote both a single molecule used as marker and a collection of identical molecules used as marker. A "marker" in the sense of this document is the combination of an affinity reagent configured to attach to an analyte and a "reporter". As such the "marker" is the virtual assignment or mapping of an affinity reagent to a particular combination of dyes (virtual marker) and the physical assembly of an affinity reagent with the combination of dyes (physical marker). In the sense of this document, the physical assembly of an affinity reagent with the combination of dyes may occur before, during, or after the introduction of the respective affinity reagent into the sample. For example when oligonucleotide sequence barcoded antibodies are used as affinity reagents, they may be brought into a sample and allowed to attach to their predetermined target structure, e.g. by physically attaching the unique combination of dyes ($\mathbf{s}_i$) to the assigned affinity reagent ($\mathbf{a}_i$), before or after introducing at least some affinity reagents from the plurality of affinity reagents ($\mathbf{A}$) to the sample or to the chemical compound or to the chemical element, or before a generation of a readout from emission light emitted by excited dyes. In an iterative staining-imaging-dye deactivation process an affinity reagent bound to a predetermined target structure may be cyclically connected to a sequence of different combinations of dyes, as in a first combination of dyes in a first iteration and a second combination of dyes in a second iteration, a strategy to which this document refers as *"Primary qualitative iterative multi-species readout volume decoding by reassigning codes in between iterations ("code swapping")".* In other words, one or more of the markers in the sample will change between iterations.

**"Reporter":** In the sense of this document "reporter" is used to denote both a single molecule/structure used as reporter and a collection of identical molecules/-structures used as reporter. A "reporter" in the sense of this document is the combination of a unique "combination of dyes" and "linker", configured to connect the combination of "dyes" with the "affinity reagent".

**"Linker":** In the sense of this document the linker denotes a unipartite chemical structure (e.g. a monomeric molecule or a polymer) or multipartite assembly of chemical structures linking a combination of fluorescent dyes to an affinity reagent. A linker might be directly or covalently coupled to the dyes and to the affinity reagent or indirectly through for example affinity tag-affinity ligand combination such as streptavidin-biotin interaction or a hapten or an oligonucleotide for example. In the case of covalent coupling this may be a site-selective coupling. Commonly used coupling chemistries such NHS-, maleimide, azide-alkine and a range of further so called click chemistries may be used to couple the linker to the affinity reagent and/or the linker to a dye. A linker may in particular comprise an oligonucleotide (e.g. DNA, RNA, LNA, PNA, morpholino, other artificial oligonucleotide), a peptide, a DNA-origami-based structure such as for example a nanoruler, a micro-/nanobead, a polymer, a micro-/nanocapsule, a micro-/nanocrystal, a carbontube, a carbon-based nanostructure (e.g. a graphene). A linker may in particular comprise an oligonucleotide and another element of the group mentioned before, for example comprise an oligonucleotide and a peptide.

**"Readout device":** In the sense of this document "readout device" refers to a device used to perform fluorescence multicolour reading or imaging. A readout device typically includes at least one excitation light source, a detection system including at least one detection channel and may as well contain filters and/or dispersive optical elements such as prisms and/or gratings to route excitation light to the sample and/or to route emission light from the sample onto to a detector or onto an appropriate area of the detector. The detection system in the sense of this document may comprise several detection channels, may be a spectral detector detecting multiple bands of the spectrum in parallel, or a hyperspectral detector detecting a contiguous part of the spectrum. The detection system comprises at least one detector, which may be a point-detector (e.g. a photomultiplier, an avalanche diode, a hybrid detector), an array-detector, a camera, hyperspectral camera. The detection system may record intensities per channel as is typically the case in cytometers or may be an imaging detection system that records images as in the case of plate readers or microscopes. A readout device with one detector channel, for example a camera or a photomultiplier, may generate readouts with multiple detection channels using, for example, different excitation and emission bands. Readout devices allow a certain number of dyes to be analysed from a given biological sample in a given run. A "run" may refer to an "iteration" or "round", i.e. the production of at least one readout for a given set of combinations of dyes and a given mapping of affinity reagents to combinations of dyes, wherein the affinity reagents are attached to the analytes. This number typically depends on the number of detection channels, **n,** the readout device is configured to provide, i.e. is able to spectrally resolve. In the case of microscopes the number of detection channels is typically 4-5 in the case of camera-based widefield detections (e.g. widefield epifluorescence microscopes, spinning disk microscopes, light sheet fluorescence microscopes), 5-12 detection channels in the case of microscopes with spectral detection concepts that typically rely on excitation or emission fingerprinting and (spectral/linear) unmixing. Hyperspectral imaging instead, which can differentiate a high number of dyes by providing very fine spectral resolution over a wide and contiguous spectral range is not yet widely deployed in microscopy. In addition to spectral properties also the lifetime of fluorescent dyes can be used to discern multiple dye species and differentiate them from autofluorescence effectively increasing the number of detection channels, **y,** which might correspond also to the maximum number of dyes in a set (i.e. excitable by the same excitation light) that can be reliably separated.

**"Oligonucleotide":** in the sense of this document refers to DNA, RNA, peptide nucleic acid, morpholino or locked nucleic acid, glycol nucleic acid, threose nucleic acid, hexitol nucleic acid or another form of artificial nucleic acid.

**"Spot":** in the sense of this document refers to a volume in the sample or region surrounding the sample, which is being read out. The size and shape of spots is dictated by the effective point spread function of the imaging system used to acquire the data.

**"Point spread function":** - In the sense of this document the term "point spread function" is used to denote the main maximum of the point spread function and unless otherwise denoted the term refers to the effective point spread function (PSF) of the imaging system, which is generally elliptical, i.e. the lateral resolution is better than the axial resolution, but may approach an almost spherical shape as more views are acquired from preferably equidistant angles.

**"Readout":** In the sense of this document the term "readout" refers to an image-based readout, which may be acquired on a microscope like a point-scanning confocal or a camera-based/widefield imaging system for example a spinning disk microscope, a light sheet fluorescence microscope, a light field microscope, a stereomicroscope. Further the term "readout" refers to non-image based readouts for example in a cytometer or a flow-through based readout device with at least one point detector or a line detector. A readout may consist of a discrete readout, for example a single acquisition of an emission spectrum or image stack, a readout may be a readout data stream, for example in a point-scanning confocal or cytometer, which is substantially continuous. Further a readout may be a sequence of images for example a spectral or hyperspectral image stack, wherein in each image fluorescence emission of different wavelength bands is recorded.

**"Readout volume":** In the sense of this document the term "readout volume" refers to the volume which is effectively detected by an optical system such as a microscope or a cytometer at a given moment in time. For systems with a continuous data stream, the "readout volume" is determined by a clock like a "pixel clock", which divides a continuous data stream into chunks that are then assigned to a certain time point or spatial location. The readout volume might depend on the effective point spread function of the imaging system, e.g. an effective point spread function might define or confine the maximum extent of a readout volume.

**"Readout sequence":** In the sense of this document the term "readout sequence" is used refer to a readout of a "readout volume" that readouts all dyes ($y_1$, $y_2$, $y_3,...y_\sigma$) in the plurality of dyes $Y_D$ from which the combinations of dyes in the plurality of combinations of dyes ($S_1$) are composed at least one time, i.e. all dyes ($y_1$, $y_2$, $y_3,...y_\sigma$) in the plurality of dyes $Y_D$ are excited at least one time and the emitted fluorescence light is detected and separated by the readout device into channels, each channel corresponding to one of the dyes ($y_1$, $y_2$, $y_3,... y_\sigma$). Such that after obtaining the readout sequence the presence or absence of a dye from the readout volume can be assessed qualitatively and/or quantitatively, wherein qualitatively refers to calling a dye present in the readout volume, when the intensity in the corresponding channel is above a certain user-defined threshold, wherein quantitatively refers to calling a dye present in the readout volume and assigning a relative intensity value or absolute number of molecules to it. The threshold may be a fixed threshold, a fixed channel-specific threshold, or a dynamically adjusted threshold. The threshold may be a combination of thresholds for example an intensity threshold and a statistical confidence in the dye separation result. The decision to call a dye present **(presence calling)** may be made dependent on passing a combination of multiple thresholds.

Preferably, a readout sequence results from exciting the sample with a first excitation light A, detecting the emitted fluorescence, and assigning it to $y_A$ channels corresponding to $Dye_{A1}$, $Dye_{A2}$, $Dye_{A3},....Dye_{Ayn}$, a second excitation light B, detecting the emitted fluorescence, and assigning it to $y_B$ channels corresponding to $Dye_{B1}$, $Dye_{B2}$, $Dye_{B3}...Dye_{Byn}$, *and repeating the process until* $Dye_{n1}, Dye_{n2}$, $Dye_{n3},...Dye_{nyn}$ (*i.e. the entire* plurality of dyes ($Y_D$) with $y_n$ members) *have been readout at least once.*

A "code" in the sense of this document is defined as follows: **S** and **T** are two finite sets, with **S** being named the "source alphabet" and **T** being named the "target alphabet". A Code

$$C: S \rightarrow T^*$$

is a total function or algorithm that uniquely represents an element from **S** as a sequence of symbols over **T.** The extension **C'** of **C**, is a homomorphism of **S\*** into **T\*,** which naturally maps each sequence of source symbols to a sequence of target symbols. In the language used in computer science a code is generally referred to as an algorithm and a sequence of symbols as an encoded string (modified from: Code. (n.d.) In Wikipedia. Retrieved June 17, 2021 from https://en.wikipedia.org/wiki/Code). In the sense of this document the finite set $S_1$ is also named the "plurality of combination of dyes", and $T_1^*$ is the finite set of strings over $T_1$ and corresponds to the "plurality of affinity reagents", which may also be named **A** or $S_2$.

Alternatively, or in addition to the encoding/decoding of combinations of dyes users may encrypt/decrypt combinations of dyes using a cipher **X**

$$X: S \longrightarrow T^*$$

Two different cases a and b are being discerned and can be regarded as different di-rectionalities of encoding/encryption.

$$S_1 \xrightarrow{C_{\alpha 1}} T_{c1}{}^* = A \quad A = S_2 \xrightarrow{C_{\beta 2}} T_{c1}{}^*$$

or or

$$S_1 \xrightarrow{X_{\alpha 1}} T_{x1}{}^* = A \quad A = S_2 \xrightarrow{X_{\beta 2}} Tx_1{}^*$$

The method disclosed in this document is compatible with both cases a and $\beta$. As well as with cases in which multiple codes $C_1$, $C_2$, $C_3$,...$C_n$ and/or ciphers $X_1$, $X_2$,$X_3$,...$X_n$ are being used so as long as they are total functions and as long as the resulting mapping is injective or bijective. In both of these cases the codes $C_1$, $C_2$, $C_3$,...$C_n$ and/or ciphers $X_1$, $X_2$, $X_3$,...$X_n$ are functions that can be inverted, i.e. decoded. In a particularly preferred embodiment of the invention, a bijective mapping (encoding or encryption) is used, which means that there is a *one-to-one correspondence* between an element ($a_i$) of the plurality of affinity reagents ($S_2$) also named ($A$) or ($T_1{}^*$) and an element ($s_i$) of the plurality of combination of dyes ($S_1$). This allows particularly easy decoding of the combination of dyes ($s_l$ to $s_k$) contained in the readout volume and thereby the identification of their associated affinity reagents ($a_l$ to $a_k$) based on a readout sequence, that assesses the presence of all dyes ($y_1$, $y_2$, $y_3$,...$y_\sigma$) in the readout volume qualitatively (presence calling, e.g. "yes"="1", "no"=0) and/or quantitatively (presence calling with relative or absolute quantitation). The microscopic examination of the readout volume as described in this document can be regarded as encoding/decoding problem, which is solved by labeling target molecules with affinity reagents that are (dynamically) linked to, and associated with, combinations of dyes, which encode those target molecules in the readout volume labeled in this way. Retrieving the identity of the target molecules which have a *one-to-one mapping* (bijective association) with the affinity reagents from the plurality of the affinity reagents is thus a decoding problem. It is important to state, that if the presence of a certain dye from the plurality of dyes has been accepted in a readout sequence based on a certain degree of statistical confidence, then that presence becomes a mathematical truth. The decoding of combinations of dyes ($s_l$ to $s_k$) is possible, when a readout sequence is observed that does not allow all possible combinations of dyes ($s_1$, $s_2$, $s_3$,...$s_n$) in the plurality of combinations of dyes ($S_1$) to be subsumed under it. In other words, if a readout sequence is observed under which all possible combinations of dyes ($s_1$, $s_2$, $s_3$,...$s_n$) in the plurality of combinations of dyes ($S_1$) can be subsumed, than one does not gain knowledge about the contents of the readout volume. This would be the case if a readout sequence would indicate that all dyes ($y_1$, $y_2$, $y_3$,...$y_\sigma$) from the plurality of dyes ($P_D$) are called "present" in the readout volume. This case is, however, unlikely even for cases in which large numbers of affinity reagents are being used, as the cardinality of the plurality of combinations of dyes ($S_1$) grows exponentially, while the number of available affinity reagents is limited to the number of targets molecules of interest. For example the entire human genome contains about 20,000 coding genes, so even if, one would use 20,000 affinity reagents in the plurality of affinity reagents to label these target molecules with a unique combination of dyes from the plurality of combination of dyes ($S_1$), it would be easy to define a plurality of dyes ($P_D$), which is large enough to ensure that the number of elements in $S_1$ >> 20,000, i.e. several orders of magnitude higher for example $10^6$ to $10^{10}$. In consequence, it is easily possible to define conditions in which the fraction $\alpha$ of actually assigned combinations of dyes to all available combinations of dyes from the plurality of combination of dyes ($S_1$) becomes very small. In this case the likelihood to observe false-positive, i.e. combinations of dyes not assigned to a marker (type I false-positive) and/or combinations of dyes assigned to an affinity reagent not physically present in the readout volume (type II false-positive) subsumable under a first readout sequences becomes lower. If the conditions are such that a single iteration does not yield satisfactory levels of statistical confidence for presence calling for any of the following: combination of dyes; affinity reagents; and target molecules contained in the readout volume, it is possible to significantly improve the analysis in different ways which will be described herein.

In a particularly preferred embodiment of the invention, a first readout sequence is acquired in a first step and the **"first set of combinations of dyes"** subsumable under this first readout sequence is stored in a memory device. In a next step for at least some affinity reagents from the plurality of affinity reagents (**A**) the encoding/encryption might be changed. This can be done by deactivating the dyes introduced in the first step by means of eluting the affinity reagents, bleaching dyes or severing the linkage between the combination of dyes and the affinity reagents. Depending on the choice of method the target molecules are then re-labeled in a second step with at least some affinity reagents from the plurality of affinity reagents (**A**), where in at least some affinity reagents are assigned to a different second combination of dyes. In a next step the **"second set of combinations of dyes"** is derived from a second readout sequence, i.e., a second readout sequence is generated in the same manner that the first readout sequence was generated and dyes from the second set of combinations of dyes identified in the second readout sequence. The retrieval of all **second combinations of dyes** subsumable under this **second readout sequence** is stored in a memory device. In a further step the **"first set of combinations of dyes"** and the **"second set of combinations of dyes"** might be compared to define the overlap and at least one statistical confidence measure is computed for each combination of dyes and/or affinity reagent and/or target molecule and/or analyte detected in the overlap. A certain combination of dyes and/or a certain affinity reagent and/or a certain target molecule and/or analyte is then said or called to be present in the readout volume, when the at least one statistical confidence measure computed for this particular certain combination of dyes and/or certain affinity reagent and/or certain target molecule and/or analyte is acceptable based on criteria, which may be fixed and a priori defined or dynamically derived and adjusted during the experiment.

In principle this process may be repeated until an acceptable level of statistical confidence in the acceptance or rejection of the presence of a certain combination of dyes and/or affinity reagents and/or target molecules and/or analytes of interest has been reached. Importantly, while in strict mathematical terms each iteration in this iterative process analyses exactly the same readout volume, it is possible and a particularly preferred embodiment of the present invention to allow small deviations (fractions 1/10000, 1/1000, 1/100, 1/10, ¼, ½ of the lateral extent of the effective PSF for example) in the spatial and/or temporal position (fractions 1/10000, 1/1000, 1/100, 1/10, ¼, ½ of the time a sample takes to traverse the lateral extent of the effective PSF for example) of the readout volume between a first and a second readout sequence. In this case a first readout sequence generates *a priori* knowledge about the second readout sequence in the sense of Bayesian probabilities according to the Bayesian theorem, this is not unlike pretest probability in diagnostic testing, in which a symptomatic patient typically has a substantially lower false-positive rate than an asymptomatic patient. In analog fashion one can argue that if an affinity reagent $\alpha_t$ was detected in a first readout volume than this influences its probability to be detected in an overlapping second readout volume, wherein the overlap may be understood as spatial or temporal.

Preferably, a "code" in the sense of this document may be for example a linear code (e.g. binary code), fixed length code, a variable length-code, or an error-correcting code. In a particularly preferred embodiment, the codes are "independent and identically-distributed". In a particularly preferred embodiment of the present invention, a binary code is used.

**"set of combinations of dyes subsumable under a readout sequence"** : In the sense of this document "set combinations of dyes subsumable under a readout sequence" refers to the set containing all combinations of dyes from the plurality of combinations of dyes that can be subsumed under a certain readout sequence. The "set of combinations of dyes subsumable under a readout sequence" subsumable under a readout sequence contains $\kappa$ elements.

**"assignment rate":** is the proportion of unique codes (also referred to as combination of dyes) from the set of unique codes, which may also be referred to as the plurality of combinations of dyes ($\boldsymbol{S}_1$), that are actually assigned to a marker and is denoted as $\alpha$.

Short Description of the Figures:

Hereinafter, specific embodiments are described referring to the drawings, wherein:

Fig. 1    shows a flow chart for a method for analysing a biological sample with a plurality of analytes,

Fig. 2    shows a schematic illustration of the method for analysing a biological sample,

Fig. 3    shows a schematic illustration on segmenting an optical readout and how the probabilistic model may be used to determine the presence of an analyte,

Fig. 4    shows an overview of a probabilistic model derived from information about the biological sample prior to generating the readout of the biological sample,

Fig. 5    shows an example of an adapted probabilistic model anchored to image data, and

Fig. 6    summarises the iteration of the probabilistic model.

Detailed Description:

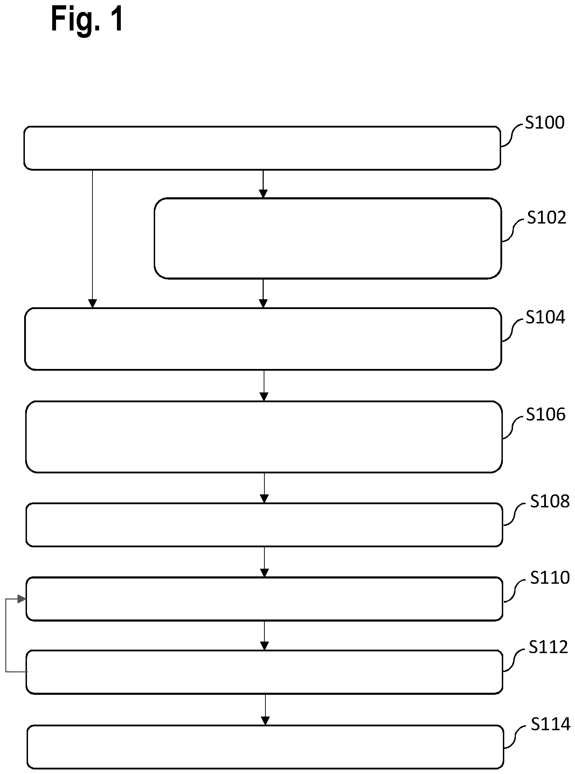

Figure 1 shows a flow chart for a method for analysing a biological sample with a plurality of analytes. In a step S100 preferably initial information about the sample is provided by the users, for example, information relating to the donor or sample origin. In particular, the sample may be a biopsy from a patient. For example, the information may be sourced from generic databases or by a clinician who generated the sample and may include the species, age, sex, electronic health record information, genetic information, treatments of the sample, or medication taken by the patient. It may also include the organ and/or site the biopsy was taken from. Further, based on the information what organ the biological sample was taken from information may be determined that describes the expected makeup of the biological sample in terms of cell types, the transcriptome of the expected cell types, or the metabolome of the expected cell type, for example. These types of information may be available from generic databases that provide transcriptomic and metabolomic data, for example, based on previously collected and analysed samples of a particular type.

Alternatively or in addition to step S100, step S102 is performed. Step S102 is also referred to as a "pre-scan", which purpose it is to generate a first optical readout or a set of image information with high speed and low cost. To this end label-free methods (brightfield microscopy, transport intensity equation, phase contrast and similar) as well as generic marker panels including chemical fluorescent stains for example DAPI or other nuclear markers, cell membrane markers, cytoskeleton markers, histone markers and optionally a limited number of markers marking proteins are suitable. This enables determining specific information about the biological sample, such as spatial information, that means information concerning which type of organelle, cell, or tissue can be found in which areas of the biological sample.

The information obtained in S100 and S102 can be quickly and cost-effectively obtained and be used in conjunction with information accessed in generic databases (e.g. HubMap, human cell atlas, PandlaoDB) or obtained by prediction to generate a first, or initial probabilistic model of the sample in step S104. This model may or may not contain spatial relationships depending on whether a "pre-scan" (S102) was performed or not.

The probabilistic model may preferably also be refined, extended, or adjusted in a further step S106 with further information from a measurement of an adjacent part of the biological sample like the next serial section, which may have been subjected to bulk or single cell sequencing. The further information may also be derived from other types of samples of the biological sample donor, such as blood drawn from the donor. Thus, further information about the biological sample may be included in the probabilistic model after its initial generation.

In step S108 an optical readout of the biological sample is generated. This is preferably generated by the iterative method described in documents PCT/EP2021/066645 and PCT/EP2021/073819; in particular, the following iterative labelling, imaging, reading out, and decoding steps may be performed until at least the presence of at least one analyte marked by a marker is determined at a sufficient level of statistical confidence:

a) providing a plurality of affinity reagents (S2), wherein each affinity reagent (a1, a2, a3,...an) of the plurality of affinity reagents (S2) is configured to specifically bind to a predetermined target structure within the biological sample or to a predetermined chemical compound or to a predetermined chemical element;

b) providing a plurality of combinations of dyes (S1) (from a plurality of dyes (YD) with $y\sigma$ members), each combination of dyes (s1, s2, s3,...sn) is unique within the plurality of combinations of dyes (S1), each combination of dyes (s1, s2, s3,...sn) comprises at least two different dyes ($|s| >= 2$);

c) wherein the plurality of combinations of dyes (S1) is composed such that each dye (y1, y2, y3,...y$\sigma$) in the plurality of combinations of dyes (S1) can be readout by a readout device; wherein dyes can be separated by a readout device into channels; each channel corresponding to one of the dyes (y1, y2, y3,...y$\sigma$); This means that each dye can be discerned from the other dyes in the same by the readout that is used for data acquisition based on any of the following properties or their combination: excitation fingerprint/spectrum, emission spectrum, intensity, fluorescence lifetime, fluorescence anisotropy, and an affinity reagent unique to the marker, the affinity reagent being configured to attach to a predetermined structure within the sample. In a particularly preferred embodiment of the invention the method disclosed in PCT/EP2021/063310 is used to substantially increase the y$\sigma$ the overall number of dyes that can be reliably discerned from each other and represented in distinct channels by a suitably configured readout device. This is particularly advantageous as even small increases in the y$\sigma$ for example from y$\sigma$STD=5 (standard widefield fluorescence microscope) without the method to y$\sigma$IHP=25 using PCT/EP2021/063310 lead to substantially higher cardinalities of the pluralities of combinations of dyes (S1) that can be generated using different functions or algorithms to compose the combinations of dyes. These substantial increases in the cardinalities of the pluralities of combinations of dyes (S1) lead to exponentially higher statistical power of the approach and are the foundation for decoding a very high number of combinations of dyes present in a readout volume with high statistical confidence in the decoding result, i.e. the presence calling.

d) preferably introducing at least some affinity reagents from the plurality of affinity reagents (A) to the sample; The plurality of affinity reagents may be introduced in entirety or in several iterative steps. In the latter case sub-pluralities of affinity reagents may be defined using a priori knowledge to optimize the conditions for presence calling.

e) before or after Step d), preferably assigning each affinity reagent of the plurality of affinity reagents (S2) to at least one combination of dyes from the plurality of combinations of dyes (S1); both the virtual assigning (virtual marker) and the physical constitution of a marker (physical marker), i.e. the physical assembly of the linkage between the affinity reagent and its assigned combinations of dyes may be performed before the introduction of the affinity reagent into the sample. In a particularly preferred embodiment of the invention, affinity reagents are introduced into the sample and allowed to attach to their predetermined target structures before the linkage between the affinity reagent and the assigned combination of dyes is established. In another particularly preferred embodiment of the invention, a linkage between an affinity reagent and its first assigned combination of dyes from a first round in an iterative process, in which steps d)-f) are repeated for at least two iterations, is severed to allow establishing of a new linkage between said affinity reagent and a second assigned combination of dyes in a second round. This may be referred to as "code swapping" or introducing a further mapping or changing the encoding of affinity reagents and/or markers, which is a powerful strategy to decode a readout volumes even if, the sample contains a very high number of different combination of dyes and associated affinity reagents.

f) preferably directing excitation light having the respective specific characteristics for exciting each dye in the plurality of combinations of dyes to the sample in order to excite the respective dyes

g) preferably generating at least one readout from emission light emitted by the excited dyes located in a readout volume of the sample by the detection channels for at least one readout volume; and

h) preferably determining which affinity reagents are present in the readout volume based on the at least one readout

In a particularly preferred embodiment the determination of the presence of affinity reagents in the readout volume is established based on a measure of statistical confidence and a certain level of statistical confidence. A measure of statistical confidence is computed for each marker and/or affinity reagent and/or combination of dyes and/or predetermined target molecule. This may be a combined measure consisting of multiple measures of statistical confidence assessing related aspects. The measure of statistical confidence may incorporate a priori knowledge and use Bayes theorem for example to adjust the probability of observing a given marker and/or affinity reagent and/or combination of dyes and/or predetermined target molecule based on a priori knowledge about that marker and/or affinity reagent and/or combination of dyes and/or predetermined target molecule. This a priori knowledge may be generated before or during the experiment as also described for steps S100, S102, S104. This means that for example a p value is computed for each marker and/or affinity reagent and/or combination of dyes and/or predetermined target molecule which assesses the probability that the detected presence (qualitative decoding) and/or quantity (relative or absolute quantitative decoding) is observed when the null hypothesis is true, i.e. the respective marker and/or affinity reagent and/or combination of dyes and/or predetermined target molecule is actually not present in the readout volume. The presence calling, i.e. the user's decision to accept the presence of a given marker and/or affinity reagent and/or combination of dyes and/or predetermined target molecule in the readout volume is then based on attaining a sufficient level of statistical confidence. The decision may be automated by using thresholds, which may be fixed and the same across all markers, affinity reagents, combinations of dyes, and target molecules or they may be different thresholds, which may be based on a priori knowledge. Further thresholds may be adjusted dynamically throughout the experiment. For example, they may be made more or less stringent. This is advantageous as it allows to demand a higher statistical confidence for target molecules, which are of particular interest.

This approach is based on viewing microscopy as an encoding/decoding problem rather than a problem of registering spatially located intensities in an image, which is essentially a matrix of intensity values. While the readout method is compatible with image-based readouts the "images" generated by the method should be regarded as probabilistic mathematical models of the reality of the sample under investigation, in which the presence of a target molecule is detected or called (presence calling) based upon the decision of the user to accept its presence based on a measure of statistical confidence and a certain level of statistical confidence in the presence of the respective target molecule in the readout volume.

Making the step of accepting the presence of a certain marker, a certain affinity reagent, and a certain target molecule based on a measure of statistical confidence and a certain level of statistical confidence (i.e. presence calling) generates a mathematical truth. This is an important aspect of the present invention as this also implies that following to presence calling one is operating in the axiomatic domain of mathematics which is inherently free of influences that complicate measurements in the non-mathematical domain, i.e. the physical, chemical, biological domain.

In step S110 at least some of the information obtained in S108 is used to update, extend, adjust, refine the first or initial probabilistic model, in particular, to generate a second probabilistic model, which now includes more of the information about the biological sample and models the actual biological sample to a higher degree.

In S112 the measurement continues and at least the presence of one additional marker is determined in at least one part of the sample, in particular, by generating a further readout as described for step S108. In the sense of this document presence may refer to a binary presence/absence decision (qualitative), semi-quantitative measurement as is typically the case for microscopy experiments or a quantitative measurement. Steps S110 and S112 are repeated iteratively until an acceptable number of markers has been measured at a sufficient level of statistical confidence or until another goal of the measurement has been attained. In step S114, the "final" mathematical probabilistic model of the sample reflecting the result of the overall experiment is generated. This final probabilistic model describes the presence of the analytes in the biological sample as determined by the method according to Figure 1.

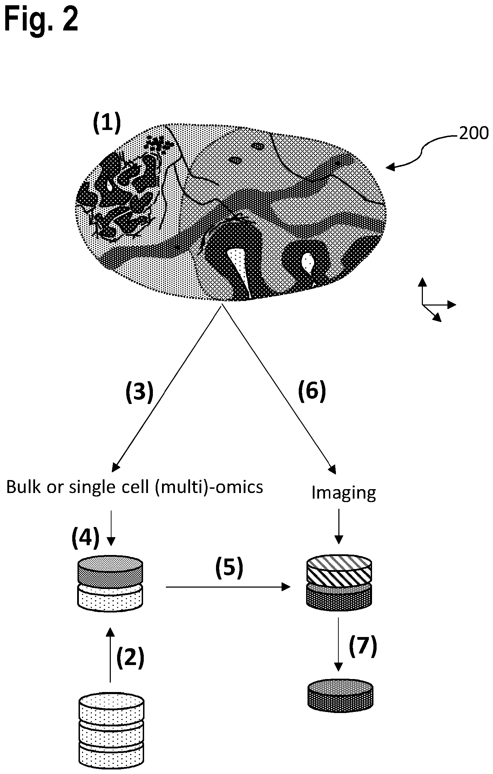

Figure 2 shows a schematic illustration of the method for analysing a biological sample. In step (1) a biopsy 200 is obtained. A suitable first set of a priori information about the sample might be obtained in step (2) from pre-existing knowledge using databases such as HubMAP, Human Cell Atlas, HTAN, and LocDB, for example. In step (3) a second set of a priori information about the sample might be obtained by analysing a part of the biopsy using bulk or single cell (multi)-omics for example whole exome sequencing. In step (4) the first and second sets may be combined and further information derived or inferred from the combination, to collectively include information on expected cell types, cell states, gene and protein expression, as well as protein localisation, for the specific biological sample 200.

In step (5) the available a priori information is used to generate an a priori probabilistic model of the biological sample 200 and optionally a recommendation is generated on the set of markers to mark those analytes expected in the biological sample that may be relevant for a particular biological question.

In step (6) the sample is stained or marked with markers preferably comprising combinatorial dyes or labels and an optical readout is generated. This may be an iterative process of imaging, blanking and a further staining step. The data input from the optical readout might then be compared to the a priori probabilistic model and region- or pixel-specific probabilities are used to improve the decoding of the combinatorial labels. Finally, a probabilistic multi-scale multi-layer mathematical model of the sample might be generated in step (7). Thus, various sources of data for different data types for example, genomic (DNA), transcriptomic (RNA), proteomic (protein, protein complexes), localisomic (subcellular localisation), metabolomic (metabolite). Interactomic (protein-protein interaction), epigenomic (DNA methylation, histone modification, chromatin accessibility), are integrated by the method and stored as a multi-scale multi-layer probabilistic model of the sample. Further, the multi-scale multi-layer probabilistic model, includes information for different levels of the biological sample such as a tissues sections, to tissue modules, certain anatomical structures, groups of cells, individual cells, subcellular compartments for example the plasma membrane, the cytosol, any of the cellular organelles (e.g. mitochondria, phagosome, endoplasmic reticulum, Golgi network), the nucleus or nucleolus. This model may be stored on a remote server, such as a cloud.

It is important to note that generally acceptable levels of statistical confidence for example acceptable p-values for the presence of a certain marker in a certain quantity in a certain location (i.e. pixel or voxel) might be attained already for a first round of the iterative process, in other words the iterations needed for performing the method described in documents PCT/EP2021/066645 and PCT/EP2021/073819 may be substantially reduced even to the point, where no iterations are needed and a single round is sufficient for practical application of the method.

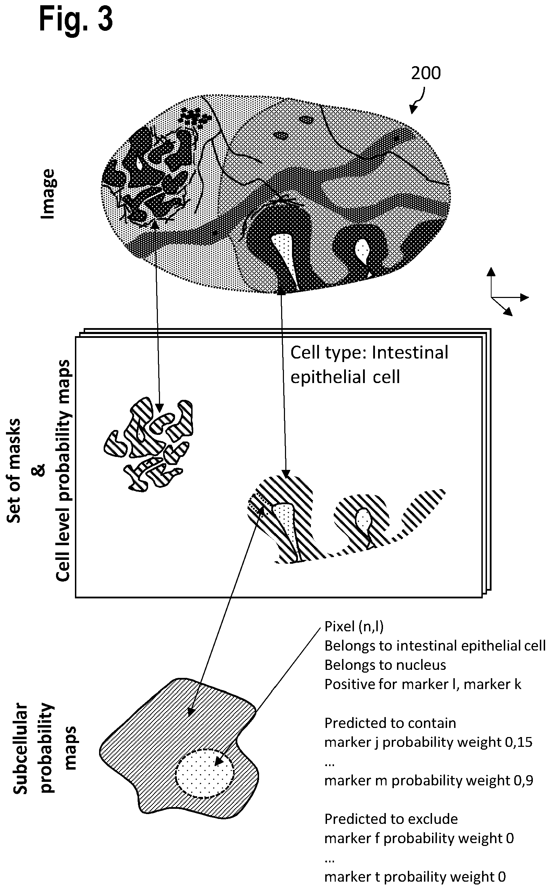

Figure 3 shows a schematic illustration on segmenting an optical readout and how the probabilistic model may be used to determine the presence of an analyte. Initially the optical readout of the biological sample 200 may be segmented to identify areas in the readout belonging to a particular tissue, a particular cell, and/or a particular subcellular area. Alternatively or in addition, a generated probabilistic model (for example, based on step (5) of Figure 2) may be used to provide predictions on the presence (probability weight >0) or absence (probability=0) of certain analytes in certain areas of the biological sample. Thus, the probabilistic model may provide a set of analytes that are expected to be found in a certain type of cell, for example, of the biological sample. This probabilistic data may be used to annotate the readout. For example, the model may provide cell-level probability maps, which enable identifying pixels of the readout as a certain type of cell based on the markers found and therefore the corresponding analytes. Similarly, on the subcellular level probability maps annotate pixels as for example "belongs to intestinal epithelial cell, belongs to nucleus..." and provide predictions on the presence of certain analytes.

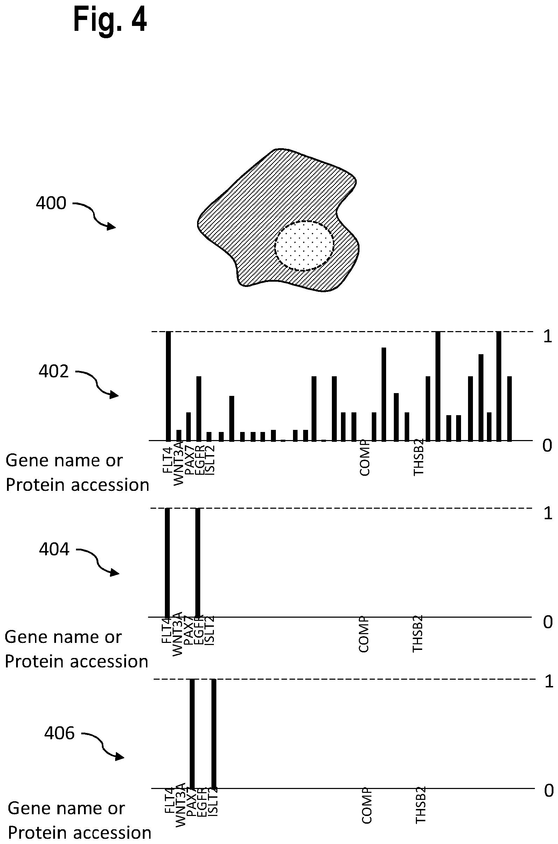

Figure 4 shows an overview of a probabilistic model derived from information about the biological sample derived prior to generating the readout of the biological sample. For example, the probabilistic model may include a model of a tissue 400, resembling an "average" that characterises a certain cluster or metastable state in a UMAP embedding or tracjectory analysis stemming from analysis of for example thousands of enterocytes by single cell RNAseq (this data may be obtained by the user, compare Figure 2 or through the human cell atlas for example). This model has multiple levels (cell-wide, nucleus, cytosol) and types (genomics, transcriptomic, proteomics, localisomic, metabolomic, ...) of information. The information contained in the model may be more or less extensive in the sense of levels as well as layers. In particular a first mathematical probabilistic model of the sample will have a lower information content and will then be progressively updated, refined, adjusted and expanded with (A) information deriving from progressive measurement (i.e. more rounds of imaging, staining, blanking, decoding yielding more markers measured at sufficient level of statistical confidence) as well as (B) more data from generic databases or information derived from the data as well as potentially data from further measurements of the sample or further measurements on further samples from the same donor. This is particularly relevant in the context of a diagnostic use of the method or a use in the context of guiding or monitoring therapy of a patient. Further measurements of the sample may be other modalities like mass spectrometry, IMC, MIBI, etc. Further measurements on further samples from the same donor may be in particular, blood measurements, liquid biopsies, MRI scans, CT scans, physiological measurements.

As an example, the model 400 includes information on probabilities that certain genes and/or proteins are expressed in the tissue in general (reference sign 402). For particular subcellular compartments, the model can comprise probabilities which genes and/or proteins are expressed in cell membranes (reference sign 404) and in the nuclei (reference sign 406).

As detailed before, the model 400 may initially be based on information from generic databases and only contain general information about the biological sample. The probabilities 402, 404, 406 may therefore initially be of only general applicability to the specific biological sample. However, by including information derived from the biological sample, for example, by using information obtained from substantially the same sample, e.g. a neighbouring serial section subjected to whole genome-sequencing and computational deconvolution of cell types, and information retrieved from databases, prediction servers, or own inference on any of the data mentioned before. Further, a "pre-scan" as described in Figure 1 S102 may be used to adjust the initial probabilistic model to obtain an adapted probabilistic model of the sample, which includes more detailed information derived from the biological sample and therefore may increase the predictive accuracy of the model.

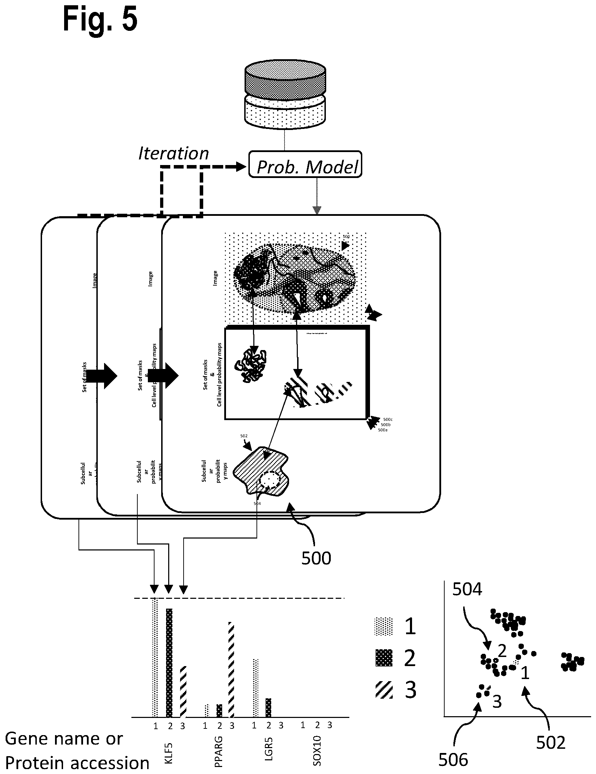

Figure 5 shows an example of an adapted probabilistic model(s) anchored to image data i.e. weights or probabilities are associated to pixels or voxels which themselves are classified as belonging to regions that are classified as belonging to a certain class in the a priori ontology like e.g. intestinal epithelium, intestinal cell, enterocyte. The illustration shows 3 probabilistic models 1, 2, 3, wherein probabilistic model 1, 2, 3 are progressively refined. This become clear by looking at a region of the sample masked or segmented and classified to be intestinal epithelium, containing a plurality of segmented cells classified as belonging to intestinal cell lineage for example, which is associated with corresponding cell-wide or "global" parameter profiles (containing probabilities or weights) a small part of such a profile is illustrated and contains 4 genes and the respective gene products e.g. proteins. In this example the genes are KLF5, PPARG, LGR5, SOX10. As depicted in Figure 5 this meta-intestinal cell profile includes "0" weights/probabilities for SOX10 as this marker is known not to be expressed in this cell lineage as well as weights > 0 for LGR5 a marker of intestinal stem cells as well as PPARG and KLF5, which are both strongly expressed and are biomarkers with good sensitivity for this cell lineage. The latter of which KLF5 also displays good specificity, i.e. it tends to not be expressed in cells of other lineages. As depicted in Figure 5 with progressive measurements, which may or may not be in synchrony with the iterations performed in steps S108 and S112 (i.e. additional rounds of staining, imaging, blanking, decoding), more markers are measured at sufficient level of statistical confidence. This additional information is then used to adjust the mathematical models. For example more information on the presence of further markers may be used to adjust, extend, refine, improve a UMAP clustering or trajectory analysis underlying and there change the ontology or change the assignment of a certain cell in the sample to a certain cluster. In the present example in the probabilistic model 1 a cell 500 was classified as an intestinal cell and associated with the appropriate meta-cell type the profile was adjusted with the markers that were measured and the probabilistic model 2 was generated, wherein the re-clustering re-assigned this particular cell 500 to a different cluster. For example, it was reassigned from the intestinal stem cell cluster 502 to an intestinal progenitor cluster 504 as more information on differentiation markers became available. In the probabilistic model 3 the cell 500 was re-assigned to a re-clustered cluster 506, which is assigned to meta enterocyte.

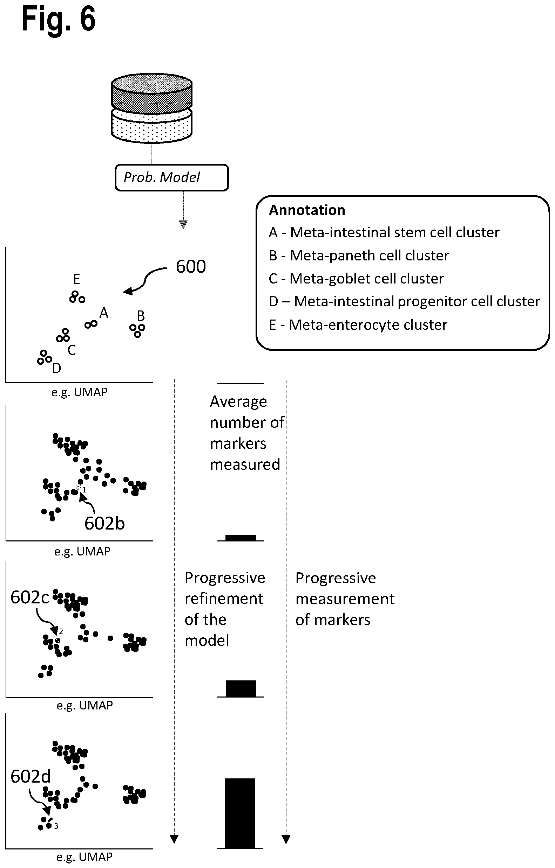

Figure 6 summarises the iteration of the probabilistic model starting from a non-spatial meta-tissue model 600 containing multiple meta cells (white dots) per meta cell type (white dote clusters). The model might also contain just one meta cell per meta cell type or multiple, for example, 10, 100, 1000, 10,000, 100,000, or more to accommodate different cell states e.g. physiological states, cell cycle states, signaling states, or simple cell-to-cell, sample-to-sample variability. As depicted in Figure 6 the non-spatial meta-tissue model may be adjusted, extended, refined, improved to a become spatial meta-tissue model by associating each dot in the for example UMAP embedding with a segmented cell in the optical readout data and preferably also by assigning each cell to a certain class. The information obtained in the "pre-scan" should be sufficient to segment most of the cells in the tissue such that the UMAP embedding in the example now contains much more dots representing cells for which a probabilistic model exists that has at least one adjustment with information obtained from the "pre-scan" or main measurement. In the example this is represented by black dots to distinguish them from the purely generic models represented by white dots. As illustrated progressive measurement yields more markers, whose presence has been determined with sufficient statistical confidence per cell on average, which allows progressive refinement of the model, which can be seen by the slight changes schematically depicted for the UMAP embedding, which represents the same cell 500 at different positions in different embeddings 602b, 602c, 602d. Based on the analysis of multiple markers and the change of the embedding from one cycle to the next one can derive a measure of statistical confidence in the clustering result, which can also be regarded cell type or cell state calling. I.e. if steps would be repeated and cell 500 would stay substantially in the same position and same cluster, then the confidence in the assignment to the cluster would be higher and then the annotation of the cluster based on the characterisation as a certain cell type e.g. enterocyte can be regarded as cell type calling. The same applies to cell state and also different organizational levels such as tissue type, tissue state for example. Based on such a clustering a tissue section might be identified as belonging to an adult male donor, gut, intestinal epithelium, inflammatory tissue state.

Example for determining the presence of an analyte:

All possible a-priori information leads to two probability measures that are relevant for determining the presence of analytes and/or associated markers (for example marked proteins) in the readout volume.

1. Localization probability to find a specific analyte $m_j$ in the readout volume:

$$P_j = P(m_j)$$

2. Co-localization probability to find two analytes in the same readout volume:

$$P_{ij} = P(m_i|m_j)$$

Let us consider that K possible combinations of the analytes are detected in a readout volume. For each of the combinations, the total probability can be calculated as

$$P_K = \prod_{ij \in \Omega_K} P(m_i)P(m_i|m_j)$$

with the set of the possible proteins $\Omega_K$. By setting the threshold value at 0.1%, the number of the possible combinations can drastically be decreased. Note that it is also possible to consider higher correlation orders such as $P_{ijk} = P(m_i|m_j|m_k)$.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

List of Reference Signs

| | |
|---|---|
| 200 | Biological sample |
| 400, 600 | Probabilistic model |
| 402 | Tissue expression |
| 404 | Cell membrane expression |
| 406 | Nucleic expression |
| 500 | Cell |
| 502, 504, 506 | Cell clusters |
| 602b, 602c, 602d | Embeddings |

**Claims**

1. A method for analysing a biological sample (200) with a plurality of analytes, comprising the following steps:

   determining information about the biological sample (200) and its analytes, the analytes being marked by a plurality of markers,
   generating a probabilistic model (400, 600) of a distribution of the analytes within the biological sample (200) based on the determined information,
   generating at least one optical readout of the biological sample (200), and
   determining the presence of at least one analyte in the readout based at least partially on the probabilistic model (400, 600) of the distribution of the analytes within the biological sample (200),
   wherein the probabilistic model (400, 600) comprises for each analyte the localisation-probability that the analyte is found in a particular location of the biological sample (200) and/or the co-localisation-probability that the analyte is found in proximity with another one of the analytes, and
   **characterized in that** the localisation and/or co-localisation probabilities of the probabilistic model (400, 600) are used to distinguish between optical signals of the markers that mark the analytes in order to determine the presence of the at least one analyte in the optical readout.

2. The method according to claim 1, wherein the information about the sample (200) is determined from at least one of the following sources: generic databases or data generated from the biological sample (200).

3. The method according to one of the preceding claims, wherein the information about the sample (200) comprises at least one of the following types: genomic, transcriptomic, proteomic, metabolomic, interactomic, localisomic, or epigenomic.

4. The method according to one of the preceding claims, wherein the information about the sample (200) is determined for at least one of the following levels: subcellular, organellular, cellular, or tissular.

5. The method according to one of the preceding claims, wherein the information about the sample (200) is at least determined prior to generating the one optical readout.

6. The method according to one of the preceding claims, wherein the at least one optical readout is segmented in order to determine the information about the sample from the segmented optical readout.

7. The method according to one of the preceding claims, wherein the information about the sample is determined from the optical readout pixel-per-pixel.

8. The method according to one of the preceding claims, wherein the probabilistic model is modified based on information about the biological sample (200) determined from the at least one optical readout, preferably, the probabilistic model is iteratively modified based on information about the biological sample determined from a plurality of generated optical readouts.

9. The method according to one of the preceding claims, wherein at least one further optical readout is generated and the analytes are marked by a further plurality of markers for the further optical readout.

10. The method according to one of the preceding claims, wherein at least the plurality of markers is based on the probabilistic model of the distribution of the analytes.

11. The method according to one of the preceding claims, wherein generating the optical readout comprises:

   i) providing the plurality of markers comprising a plurality of affinity reagents, each affinity reagent configured to attach to one of the analytes, and a first plurality of combinations of dyes, each combination of dyes being unique within the first plurality of combinations of dyes and each combination of dyes comprising at least two dyes having different characteristics for at least one of: excitation and emission, wherein each one of the unique combinations of dyes is attached to an associated affinity reagent according to a first mapping,
   ii) directing excitation light at the sample, the excitation light having characteristics for exciting at least the at least two dyes having different characteristics for at least one of: excitation and emission, and
   iii) generating at least one first optical readout from emission light emitted by the excited dyes.

12. The method according to claim 11, further comprising at least one of:

   deactivating at least one of the dyes in the first plurality of combinations of dyes,
   removing the attachment between at least one affinity reagent and at least one of the combinations of dyes,
   removing the attachment between at least one affinity reagent and at least one of the analytes, and
   waiting longer than a fluorescence lifetime of at least one of the dyes in the first plurality of combinations of dyes; and
   repeating steps i) to iii) of claim 11 for a second, different, plurality of combinations of dyes or for the first plurality of combinations of dyes according to a second, different, mapping.

13. The method according to claim 12, further comprising:
   providing at least one dye and/or dye combination of dyes for the second plurality of combinations of dyes and/or rules for the second mapping, based on the at least one first optical readout.

14. The method according to claims 12 or 13, further comprising iteratively repeating the steps of claim 12 and/or claim 13 for at least one of: a number of pluralities of combinations of dyes; or a number of mappings, until all affinity reagents attached to analytes in the sample are determined.

**Patentansprüche**

1. Verfahren zur Analyse einer biologischen Probe (200) mit einer Vielzahl von Analyten, umfassend die folgenden Schritte:

   Bestimmen von Informationen über die biologische Probe (200) und ihre Analyten, wobei die Analyten durch eine Vielzahl von Markern markiert sind,
   Erzeugen eines Wahrscheinlichkeitsmodells (400, 600) einer Verteilung der Analyten innerhalb der biologischen Probe (200) auf der Grundlage der bestimmten Informationen,
   Erzeugen mindestens einer optischen Auslesung der biologischen Probe (200), und
   Bestimmen des Vorhandenseins mindestens eines Analyten in der Auslesung mindestens teilweise auf der Grundlage des Wahrscheinlichkeitsmodells (400, 600) der Verteilung der Analyten innerhalb der biologischen Probe (200),
   wobei das Wahrscheinlichkeitsmodell (400, 600) für jeden Analyten die Lokalisierungswahrscheinlichkeit umfasst, dass der Analyt an einem bestimmten Ort der biologischen Probe (200) gefunden wird, und/oder die Ko-Lokalisierungswahrscheinlichkeit, dass der Analyt in der Nähe eines anderen der Analyten gefunden wird, und

**dadurch gekennzeichnet, dass**

die Lokalisierungs- und/oder Ko-Lokalisierungswahrscheinlichkeiten des Wahrscheinlichkeitsmodells (400, 600) verwendet werden, um zwischen optischen Signalen der Marker, die die Analyten markieren, zu unterscheiden, um das Vorhandensein des mindestens einen Analyten in der optischen Auslesung zu bestimmen.

2. Verfahren nach Anspruch 1, wobei die Informationen über die Probe (200) aus mindestens einer der folgenden Quellen bestimmt werden: generische Datenbanken oder aus der biologischen Probe (200) erzeugte Daten.

3. Verfahren nach einem der vorstehenden Ansprüche, wobei die Informationen über die Probe (200) mindestens einen der folgenden Typen umfassen: Genomik, Transkriptomik, Proteomik, Metabolomik, Interaktomik, Lokalisomik oder Epigenomik.

4. Verfahren nach einem der vorstehenden Ansprüche, wobei die Informationen über die Probe (200) für mindestens eine der folgenden Ebenen bestimmt werden: subzellulär, organellulär, zellulär oder gewebeartig.

5. Verfahren nach einem der vorstehenden Ansprüche, wobei die Informationen über die Probe (200) mindestens vor der Erzeugung der einen optischen Auslesung bestimmt werden.

6. Verfahren nach einem der vorstehenden Ansprüche, wobei die mindestens eine optische Auslesung segmentiert wird, um die Informationen über die Probe aus der segmentierten optischen Auslesung zu bestimmen.

7. Verfahren nach einem der vorstehenden Ansprüche, wobei die Informationen über die Probe aus der optischen Auslesung Pixel für Pixel bestimmt werden.

8. Verfahren nach einem der vorstehenden Ansprüche, wobei das Wahrscheinlichkeitsmodell auf der Grundlage von Informationen über die biologische Probe (200) modifiziert wird, die aus der mindestens einen optischen Auslesung bestimmt werden, vorzugsweise wird das Wahrscheinlichkeitsmodell iterativ auf der Grundlage von Informationen über die biologische Probe modifiziert, die aus einer Vielzahl von erzeugten optischen Auslesungen bestimmt werden.

9. Verfahren nach einem der vorstehenden Ansprüche, wobei mindestens eine weitere optische Auslesung erzeugt wird und die Analyten durch eine weitere Vielzahl von Markern für die weitere optische Auslesung markiert werden.

10. Verfahren nach einem der vorstehenden Ansprüche, wobei mindestens die Vielzahl der Marker auf dem Wahrscheinlichkeitsmodell der Verteilung der Analyten basiert.

11. Verfahren nach einem der vorstehenden Ansprüche, wobei das Erzeugen der optischen Auslesung umfasst:

i) Bereitstellen der Vielzahl von Markern, die eine Vielzahl von Affinitätsreagenzien umfassen, wobei jedes Affinitätsreagenz so konfiguriert ist, dass es an einen der Analyten bindet, und eine erste Vielzahl von Farbstoffkombinationen, wobei jede Farbstoffkombination innerhalb der ersten Vielzahl von Farbstoffkombinationen einzigartig ist und jede Farbstoffkombination mindestens zwei Farbstoffe mit unterschiedlichen Eigenschaften für mindestens eines umfasst von: Anregung und Emission, wobei jede der einzigartigen Farbstoffkombinationen an ein zugehöriges Affinitätsreagenz entsprechend einem ersten Mapping gebunden ist, ii) Richten von Anregungslicht auf die Probe, wobei das Anregungslicht Eigenschaften zur Anregung von mindestens den mindestens zwei Farbstoffen mit unterschiedlichen Eigenschaften für mindestens eines aufweist von: Anregung und Emission, und iii) Erzeugen mindestens einer ersten optischen Auslesung aus dem von den angeregten Farbstoffen emittierten Emissionslicht.

12. Verfahren nach Anspruch 11, weiter umfassend mindestens eines von:

Deaktivieren mindestens eines der Farbstoffe in der ersten Vielzahl von Farbstoffkombinationen, Entfernen der Bindung zwischen mindestens einem Affinitätsreagenz und mindestens einer der Farbstoffkombinationen, Entfernen der Bindung zwischen mindestens einem Affinitätsreagenz und mindestens einem der Analyten, und Warten länger als eine Fluoreszenzlebensdauer von mindestens einem der Farbstoffe in der ersten Vielzahl von Farbstoffkombinationen; und Wiederholen der Schritte i) bis iii) von Anspruch 11 für eine zweite, unterschiedliche Vielzahl von Farbstoffkombinationen oder für die erste Vielzahl von Farbstoffkombinationen entsprechend einem zweiten, unterschiedlichen Mapping.

13. Verfahren nach Anspruch 12, weiter umfassend: Bereitstellen mindestens eines Farbstoffs und/oder einer Farbstoffkombination von Farbstoffen für die zweite Vielzahl von Farbstoffkombinationen und/oder Regeln für das zweite Mapping, basierend auf der mindestens einen ersten optischen Auslesung.

14. Verfahren nach Anspruch 12 oder 13, weiter umfassend das iterative Wiederholen der Schritte von Anspruch 12 und/oder Anspruch 13 für mindestens eines von: einer Anzahl von Vielzahlen von Farbstoffkombinationen; oder einer Anzahl von Mappings, bis alle an Analyten in der Probe gebundenen Affinitätsreagenzien bestimmt sind.


**Revendications**

1. Procédé d'analyse d'un échantillon biologique (200) avec une pluralité d'analytes, comprenant les étapes suivantes :

   la détermination d'informations concernant l'échantillon biologique (200) et ses analytes, les analytes étant marqués par une pluralité de marqueurs,
   la génération d'un modèle probabiliste (400, 600) d'une distribution des analytes au sein de l'échantillon biologique (200) sur la base des informations déterminées,
   la génération d'au moins une lecture optique de l'échantillon biologique (200), et
   la détermination de la présence d'au moins un analyte dans la lecture sur la base au moins en partie du modèle probabiliste (400, 600) de la distribution des analytes au sein de l'échantillon biologique (200),
   dans lequel le modèle probabiliste (400, 600) comprend pour chaque analyte la probabilité de localisation que l'analyte soit dans un emplacement particulier de l'échantillon biologique (200).
   et/ou la probabilité de co-localisation que l'analyte soit à proximité d'un autre des analytes, et **caractérisé en ce que**
   les probabilités de localisation et/ou de co-localisation du modèle probabiliste (400, 600) sont utilisées pour distinguer des signaux optiques des marqueurs qui marquent les analytes afin de déterminer la présence de l'au moins un analyte dans la lecture optique.

2. Procédé selon la revendication 1, dans lequel les informations concernant l'échantillon (200) sont déterminées à partir d'au moins l'une des sources suivantes : des bases de données génériques ou des données générées à partir de l'échantillon biologique (200).

3. Procédé selon l'une des revendications précédentes, dans lequel les informations concernant l'échantillon (200) comprennent au moins l'un des types suivants : génomique, transcriptomique, protéomique, métabolomique, interactomique, localisomique ou épigénomique.

4. Procédé selon l'une des revendications précédentes, dans lequel les informations concernant l'échantillon (200) sont déterminées pour au moins l'un des niveaux suivants : subcellulaire, organellulaire, cellulaire ou tissulaire.

5. Procédé selon l'une des revendications précédentes, dans lequel les informations concernant l'échantillon (200) sont au moins déterminées avant de générer ladite lecture optique.

6. Procédé selon l'une des revendications précédentes, dans lequel l'au moins une lecture optique est segmentée afin de déterminer les informations concernant l'échantillon à partir de la lecture optique segmentée.

7. Procédé selon l'une des revendications précédentes, dans lequel les informations concernant l'échantillon sont déterminées à partir de la lecture optique pixel par pixel.

8. Procédé selon l'une des revendications précédentes, dans lequel le modèle probabiliste est modifié sur la base d'informations concernant l'échantillon biologique (200) déterminées à partir d'au moins une lecture optique, préférablement, le modèle probabiliste est modifié de manière itérative sur la base d'informations concernant l'échantillon biologique déterminées à partir d'une pluralité de lectures optiques générées.

9. Procédé selon l'une des revendications précédentes, dans lequel au moins une autre lecture optique est générée et les analytes sont marqués par une autre pluralité de marqueurs pour l'autre lecture optique.

10. Procédé selon l'une des revendications précédentes, dans lequel au moins la pluralité de marqueurs est basée sur le modèle probabiliste de la distribution des analytes.

11. Procédé selon l'une des revendications précédentes, dans lequel la génération de la lecture optique comprend :

i) la fourniture de la pluralité de marqueurs comprenant une pluralité de réactifs d'affinité, chaque réactif d'affinité étant configuré pour s'attacher à l'un des analytes, et une première pluralité de combinaisons de colorants, chaque combinaison de colorants étant unique au sein de la première pluralité de combinaisons de colorants et chaque combinaison de colorants comprenant au moins deux colorants présentant des caractéristiques différentes pour au moins l'un parmi : l'excitation et l'émission, dans lequel chacune des combinaisons uniques de colorants est attachée à un réactif d'affinité associé selon un premier mappage,

ii) le fait de diriger la lumière d'excitation vers l'échantillon, la lumière d'excitation présentant des caractéristiques permettant d'exciter au moins les au moins deux colorants présentant des caractéristiques différentes pour au moins l'un parmi : l'excitation et l'émission, et

iii) la génération d'au moins une première lecture optique à partir de la lumière d'émission émise par les colorants excités.

12. Procédé selon la revendication 11, comprenant en outre au moins l'un parmi :

la désactivation d'au moins un des colorants dans la première pluralité de combinaisons de colorants,

la suppression de l'attachement entre au moins un réactif d'affinité et au moins l'une des combinaisons de colorants,

la suppression de l'attachement entre au moins un réactif d'affinité et au moins l'un des analytes, et

le fait d'attendre plus longtemps qu'une durée de vie de fluorescence d'au moins un des colorants dans la première pluralité de combinaisons de colorants ; et

la répétition des étapes i) à iii) de la revendication 11 pour une seconde pluralité différente de combinaisons de colorants ou pour la première pluralité de combinaisons de colorants selon un second mappage différent.

13. Procédé selon la revendication 12, comprenant en outre :
la fourniture d'au moins un colorant et/ou une combinaison de colorants pour la seconde pluralité de combinaisons de colorants et/ou de règles pour le second mappage, sur la base de l'au moins une première lecture optique.

14. Procédé selon les revendications 12 ou 13, comprenant en outre la répétition itérative des étapes de la revendication 12 et/ou de la revendication 13 pour au moins l'un parmi : un certain nombre de pluralités de combinaisons de colorants ; ou un certain nombre de mappages, jusqu'à ce que tous les réactifs d'affinité attachés aux analytes dans l'échantillon soient déterminés.

# Fig. 1

S100

S102

S104

S106

S108

S110

S112

S114

# Fig. 2

# Fig. 3

200

Image

Set of masks & Cell level probability maps

Cell type: Intestinal epithelial cell

Subcellular probability maps

Pixel (n,l)
Belongs to intestinal epithelial cell
Belongs to nucleus
Positive for marker l, marker k

Predicted to contain
marker j probability weight 0,15
...
marker m probability weight 0,9

Predicted to exclude
marker f probability weight 0
...
marker t probaility weight 0

# Fig. 4

Fig. 5

# Fig. 6

*Prob. Model*

600

**Annotation**
A - Meta-intestinal stem cell cluster
B - Meta-paneth cell cluster
C - Meta-goblet cell cluster
D – Meta-intestinal progenitor cell cluster
E - Meta-enterocyte cluster

e.g. UMAP

602b

e.g. UMAP

602c

e.g. UMAP

602d

e.g. UMAP

Average number of markers measured

Progressive refinement of the model

Progressive measurement of markers

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021166380 A1
- EP 2021066645 W
- EP 2021073819 W
- EP 2021063310 W
- EP 2021087551 W
- EP 2021087558 W

**Non-patent literature cited in the description**

- **RODRIGUEZ et al.** *Trends Biochem Sci.*, February 2017, vol. 42 (2), 111-129
- **YAN et al.** *Microchimica Acta*, vol. 186, 583
- **IRAVANI** ; **VARMA**. *Environ Chem Lett*, 10 March 2020
- **KACENAUSKAITE et al.** *J. Am. Chem. Soc.*, 2021, vol. 143, 1377-1385